# EUROPÄISCHE PATENTANMELDUNG

(11) **EP 4 793 356 A1**
(43) Veröffentlichungstag der Anmeldung: **19.08.2026**
(21) Anmeldenummer: 25158307.6
(22) Anmeldetag: 17.02.2025
(51) Int. Cl.: C12P 5/02, C12N 1/20, C12N 1/38

(54) **MEHRKOMPONENTEN-ZUBEREITUNG FÜR DAS VERGÄREN VON ORGANISCHEN RESTSTOFFEN**

(71) Anmelder: Bio-Additive.de GmbH, 22926 Ahrensburg (DE)
(72) Erfinder: Höfermann, Michael, 22952 Lütjensee (DE); Gatz, Sara, 07407 Rudolstadt (DE)
(74) Vertreter: Zeitler Volpert Kandlbinder Patentanwälte

(57) **Zusammenfassung**

Die Erfindung betrifft eine Mehrkomponenten-Zubereitung für die Zugabe beim Vergären von organischen Reststoffen in ein Fermentermedium, insbesondere unter anaeroben Bedingungen, insbesondere zum Erzeugen von Biogas, gemäß dem Oberbegriff des Patentanspruchs 1. Die Erfindung betrifft weiterhin ein Verfahren zum Erzeugen von Biogas, wobei organische Reststoffe in einen Fermenter gegeben und dort anaerob vergärt werden, gemäß dem Oberbegriff des Patentanspruchs 6.

## Beschreibung

Die Erfindung betrifft eine Mehrkomponenten-Zubereitung für die Zugabe beim Vergären von organischen Reststoffen in ein Fermentermedium, insbesondere unter anaeroben Bedingungen, insbesondere zum Erzeugen von Biogas, gemäß dem Oberbegriff des Patentanspruchs 1. Die Erfindung betrifft weiterhin ein Verfahren zum Erzeugen von Biogas, wobei organische Reststoffe in einen Fermenter gegeben und dort anaerob vergärt werden, gemäß dem Oberbegriff des Patentanspruchs 6.

Biogas wird durch die anaerobe Vergärung von organischen Materialien wie Pflanzenresten, Lebensmittelabfällen, tierischen Exkrementen und anderen Biomassen erzeugt. Die Biogaserzeugung basiert dabei auf folgenden grundlegenden Schritten:
Zunächst wird Biomasse gesammelt und vorbereitet. Hierzu werden organische Abfälle gesammelt und zerkleinert, um die Oberfläche für die Mikroorganismen zu vergrößern.

Anschließend erfolgt eine anaerobe Vergärung, bei der die vorbereitete Biomasse in einen Fermenter gegeben wird, wo sie unter Ausschluss von Sauerstoff durch Mikroorganismen abgebaut wird. Dieser Prozess findet in nachfolgend erläuterten Phasen statt. Zunächst erfolgt eine Hydrolyse, bei der. Komplexe organische Stoffe in einfachere Moleküle wie Zucker und Aminosäuren zerlegt werden. Diese Moleküle werden mittels Acidogenese weiter zu flüchtigen Fettsäuren, Wasserstoff und Kohlendioxid abgebaut. Die flüchtigen Fettsäuren werden in einer nachfolgenden, als Acetogenese bezeichneten Phase in Essigsäure, Wasserstoff und Kohlendioxid umgewandelt. In einer abschließenden Phase, die Methanogenese genannt wird, wandeln methanogene Archaeen (Urbakterien) die Essigsäure und Wasserstoff in Methan und Kohlendioxid um. Parallel wird Kohlenstoffdioxid und Wasserstoff in Methan und Wasser umgesetzt.

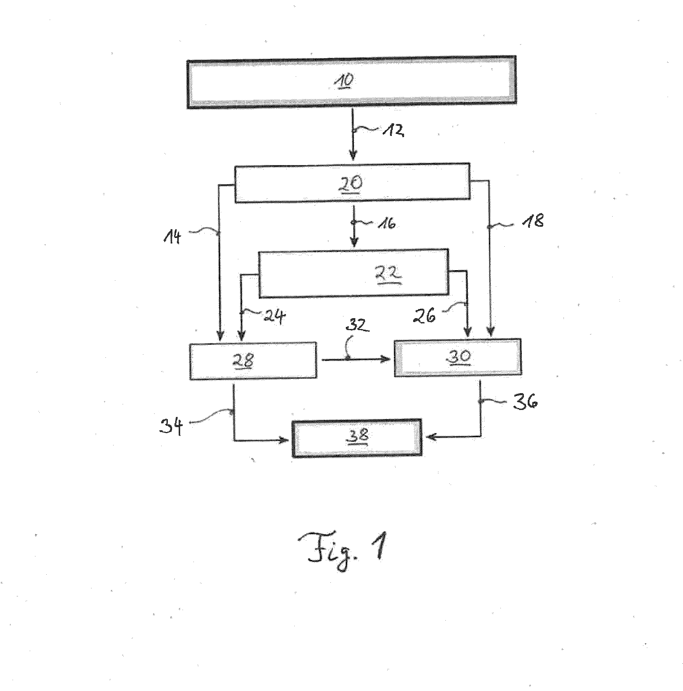

Die Methanogenese der sogenannten "anaeroben Nahrungskette" ist in Fig. 1 in einem schematischen Blockdiagramm dargestellt. Hierin steht ein erster Block 10 für Biopolymere, wie Polysaccharide und Proteine, sowie Lipide. Ein erster Pfad 12, ein zweiter Pfad 14, ein dritter Pfad 16 und ein vierter Pfad 18 stehen jeweils für einen anaeroben Gärer bzw. anaeroben Gärprozess, welcher heterotrophe Anaerobier verwendet. Ein zweiter Block 20 steht für Oligomere, Monomere. Ein dritter Block 22 steht für kurze Fettsäuren (Butyrat, Propionat,...), Alkohole. Ein fünfter Pfad 24 und eine sechster Pfad 26 stehen jeweils für syntrophe Bakterien. Ein vierter Block 28 steht für CO₂, H₂,HCOOH. Ein fünfter Block 30 steht für Acetat. Ein siebter Pfad 32 steht für acetogene Bakterien. Ein achter Pfad 34 steht für hydrogenotrophe Methanogene. Ein neunter Pfad 36 steht für acetoklastische Methanogene. Ein sechster Block 38 steht für CH₄, CO₂.

In dieser Kette werden zunächst Biopolymere, wie Proteine und insbesondere Polysaccharide wie Cellulose, über Oligomere in Monomere (beispielsweise Aminosäuren und Monosaccharide) gespalten. Lipide werden hierbei in ihre Komponenten (beispielsweise Fettsäuren) abgebaut. Danach vergären Bakterien diese Spaltprodukte zu einfachen Carbonsäuren (wie Formiat, Acetat, Propionat, Lactat und Succinat), zu Alkoholen (wie Ethanol, 2-Propanol und Butanol) und zu anderen niedermolekularen Verbindungen (H₂, CO₂ und kurzkettige Ketone). Syntrophe, acetogene Bakterien nutzen einen Teil dieser Verbindungen und setzen sie zu Acetat und C1-Verbindungen um. Im letzten Teil der anaeroben Nahrungskette, der Methanogenese, werden diese Verbindungen als Kohlenstoff-, Reduktans und Energiequelle verwendet, wobei CH₄ und meistens CO₂ freigesetzt werden.

Das erzeugte Biogas, das hauptsächlich Methan (CH₄) und Kohlendioxid (CO₂) beinhaltet, kann zur Strom- und Wärmeerzeugung genutzt werden. Es kann auch gereinigt und als Kraftstoff verwendet werden.

Die nach der Vergärung übrigbleibenden festen und flüssigen Rückstände werden als Gärreste bezeichnet. Diese können beispielsweise als Dünger in der Landwirtschaft verwendet werden.

Die anaerobe Vergärung aus organischen Abfällen hat weltweit Aufmerksamkeit erregt, um die Treibhausgasemissionen zu reduzieren, die Verbrennung fossiler Brennstoffe zu verringern und eine nachhaltige Versorgung mit erneuerbaren Energien zu ermöglichen. Biogas besteht hauptsächlich aus Methan (CH₄) (50 bis 75%), Kohlendioxid (CO₂) (25 bis 50 %), Schwefelwasserstoffe (H₂S), Wasserstoff (H₂), Ammoniak (NH₃) (1 bis 2 %) und Spuren anderer Gase wie Sauerstoff (O₂) und Stickstoff (N₂). Methan kann fossile Brennstoffe in verschiedenen Anwendungen ersetzen, beispielsweise bei der Wärme- und Stromerzeugung oder im Verkehrssektor. Der Abbau von organischen Abfällen durch ein Vergärungsverfahren bietet viele Vorteile, wie z.B. die Verringerung von Krankheitserregern und die Verhinderung der Geruchsfreisetzung. Die Gärreste aus der anaeroben Vergärung sind ein wertvoller Dünger, jedoch ist die Menge an organischen Stoffen, die derzeit für die Biogasproduktion zur Verfügung stehen, noch begrenzt. Neue Substrate sowie effektivere Umwandlungstechnologien werden benötigt, um diese Branche weltweit wachsen zu lassen.

Technologien, die darauf abzielen, die Effizienz und Nachhaltigkeit der Vergärungsprozesse zu verbessern sind vielfältig und einige bisher bekannte Aspekte betreffen folgendes:
Eine Optimierung der Biogasproduktion wird durch moderne Anlagen erzielt, die fortschrittliche Steuerungssysteme und Sensorik nutzen, um die Biogasproduktion zu maximieren.

Zur verbesserten Substratvorbehandlung werden Techniken wie die thermische Hydrolyse oder mechanische Zerkleinerung eingesetzt, um die Abbaubarkeit der organischen Materialien zu erhöhen und die Methanausbeute zu steigern.

Für die Optimierung des biologischen Abbauprozesses ist der Einsatz von Zusatzstoffen vorgesehen, die einen positiven Effekt auf die Leistungsfähigkeit der Mikroorganismen im System haben sollen.

Eine effiziente Gärrestverwertung wird durch den Einsatz von Technologien zur Nachbehandlung der Gärreste erzielt, die zur besseren Nutzung der Nebenprodukte beiträgt und die Umweltbelastung reduziert.

Zur Automatisierung und Digitalisierung ist der Einsatz von Automatisierungstechnologien und digitalen Plattformen vorgesehen, so dass eine präzisere Steuerung und Überwachung der Prozesse ermöglicht wird, was zu einer höheren Effizienz und Zuverlässigkeit einer entsprechenden Anlagen führt.

Während bei der Verbesserung der Biogasanlagen bisher viel Aufmerksamkeit der technischen und mechanischen Vorbehandlung der Biomasse und der anaeroben CO-Vergärung (in dem Sinne, dass zusätzliche organische Reststoffe aus anderen Branchen mit genutzt werden) gewidmet wird, gibt es keine wesentlichen Bemühungen hinsichtlich der Einführung von Zusatzstoffen in das Fermentermedium, welche auf molekularer Basis arbeiten.

Die Optimierung des biologischen Abbauprozesses mit Hilfe von verschiedenen Additiven ist im landwirtschaftlichen Bereich gängige Praxis. Hier kommen als Substrate in der Regel Wirtschaftsdünger wie Gülle und Mist und verschiedene Silagen (Mais, Getreide usw.) zum Einsatz. In Biogasanlagen, die organische Abfallstoffe (z.B. aus der Lebensmittelindustrie) einsetzten, kommt es hierbei jedoch besonders häufig zu Störungen (Schaumbildung, Übersäuerung, verringerte Methanbildung). Ursache dafür sind die starken Schwankungen in der Zusammensetzung und Menge des Inputmaterials sowie der Einfluss von verschiedenen Hemmstoffen.

Um eine Biogasanlage wirtschaftlich zu betreiben, ist ein möglichst stabiler und effizienter Prozess nötig. Dieser hängt vor allem von der Mikroorganismenpopulation ab, welche durch Parameter wie Substrat, Temperatur, pH-Wert, Nähr- und Hemmstoffkonzentrationen beeinflusst wird. Durch den gezielten Einsatz von Additiven kann diese Population positiv beeinflusst werden. So verringert die Zugabe von bspw. Entschwefelungsmitteln wie Eisenhydroxid die Entstehung von für die Bakterien giftigem Schwefelwasserstoff, welcher auch im Biogas nur in geringsten Mengen enthalten sein darf. Die Nutzung von Spurennährstoffen (Cobalt, Selen, Nickel, Molybdän. Mangan, und dergleichen) gleicht etwaige Mängel aus, sodass sich die Bakterien stabil vermehren und sie die Substrate effizient abbauen können. Durch den gezielten Einsatz bestimmter Enzympräparate ist es zudem möglich den ersten und für die Mikroorganismen schwierigsten Schritt, die Hydrolyse, stark zu beschleunigen. So können die großen organischen Makromoleküle in viel kürzerer Zeit gespalten und den Bakterien zur Verfügung gestellt werden. Dies bedingt eine höhere Biogasausbeute und zusätzlich die Möglichkeit mehr Substratmengen in der gleichen Zeit umzusetzen, was die erzeugte Biogasmenge noch weiter steigert.

Die anaerobe Vergärung erzeugt nachhaltige Energie aus verschiedenen organischen Reststoffen. Obwohl diese Technologie weit verbreitet ist, schränken ihre im Vergleich zum aeroben Abbau geringe biologische Abbaueffizienz und ihre schlechte Stabilität ihre kommerzielle Anwendung ein. Es hat sich gezeigt, dass die Verwendung von Additiven eine deutliche Verbesserung der Vergärungsleistung bietet. In der Praxis ist die Verwendung von Zusatzstoffen für die anaerobe Vergärung jedoch nicht vollständig verstanden. Die Verwendung von Additiven im industriellen Prozess ist aufgrund der großen Unterschiede bei den Substraten und den Betriebsverfahren bisher nicht prozesssicher möglich.

Bisher wurden zahlreiche Methoden eingesetzt, um die oben erläuterten Nachteile zu beheben, wie z. B. mehrstufigen Bioreaktortechnologie, thermische/ hydrothermale Vorbehandlung, Hochspannungs-Impulsentladung, Ultraschall, Autoklavieren, Einfrieren/Auftauen und Mikrowellenverfahren. Der Einsatz dieser Methoden ist jedoch unwirtschaftlich, da hohe Mehrkosten entstehen.

Die Verwendung von Additiven in anaeroben Reaktoren zur Förderung der Abbaurate ist ein vielversprechender Ansatz zur Bewältigung der aktuellen Probleme. Jedes Additiv bietet einzigartige Vorteile für die Förderung der Biogasproduktion in der Vergärung. Obwohl sich gezeigt hat, dass viele Additive die Biogasproduktion während des Biogasprozesses erhöhen, gibt es nur wenige umfassende Untersuchungen über den Einsatz von Additiven zur Förderung der Effizienz sauberer Bioenergie. Derzeit fehlt ein umfassendes Verständnis der verschiedenen Arten von Additiven. Insbesondere müssen die Dosierungen, Wirkungen und Anwendungen der einzelnen Additive genauer untersucht werden.

Aus der CN 114 196 654 A ist ein Verfahren zur Erzeugung von Biogas sowie eine Enzympräparat-Zusammensetzung bekannt. Diese Enzympräparat-Zusammensetzung beinhaltet folgende Rohmaterialien, 20 bis 30 Gew.-% Amylase, 20 bis 30 Gew.-% Cellulase, 15 bis 25 Gew.-% Xylanase, 5 bis 10 Gew.-% Lipase und 5 bis 10 Gew.-% Pektinase; wobei die Amylase mindestens eine aus der Gruppe α-Amylase, β-Amylase, Glucoamylase und Amylo-1,6-Glucosidase ist.

Der Erfindung liegt die Aufgabe zugrunde, die Dosierung und die Art der Additive für die industrielle anaeroben Vergärung von organischen Materialien zu verbessern.

Diese Aufgabe wird erfindungsgemäß durch eine Mehrkomponenten-Zubereitung der o.g. Art mit den in Anspruch 1 gekennzeichneten Merkmalen und durch ein Verfahren der o.g. Art mit den in Anspruch 4 angegebenen Verfahrensschritten gelöst. Vorteilhafte Ausgestaltungen der Erfindung sind in den weiteren Ansprüchen beschrieben.

Hierzu ist es bei einer Mehrkomponenten-Zubereitung der o.g. Art erfindungsgemäß vorgesehen, dass die Mehrkomponenten-Zubereitung folgende Komponenten aufweist,
- eine Spurennährstoffmischung A, die für sich betrachtet folgendes beinhaltet, 25 bis 50 Gew.-% Dinatrium-Nickel-EDTA, 5 bis 10 Gew.-%, Cobaltdinatriumethylendiamintetraacetat und 2,5 Gew.-%Natriumselenit und 37,5 bis 67,5 Gew.-% Wasser;
- ein Enzympräparat A, das für sich betrachtet folgendes beinhaltet, 5 Gew.-% einer Xylanase, 5 bis 10 Gew.-% einer Amylase, 2,5 Gew.-% einer Lipase, 5 bis 10 Gew.-% Cellulase, 1,0 bis 2,0 Gew.-% einer Glucanase, 0,1 Gew.-% Dextrose, 0,01 Gew.-% Natriumbenzoat, 0,02 Gew.-% Sorbat und 70,37 bis 81,37 Gew.-% Wasser; und
- ein Enzympräparat B, das für sich betrachtet folgendes beinhaltet, 2,5 Gew.-% einer Protease, 1,9 Gew.-% Dextrose, 0,24 Gew.-% Natriumbenzoat, 0,48 Gew.-% Sorbat, 94,88 Gew.-% Wasser.

Dies hat den Vorteil, dass der Prozess der Vergärung zeitlich beschleunigt und gleichzeitig der Vergärungsvorgang auch mit unterschiedlichsten organischen Reststoffen stabilisiert wird, d.h. bekannte Probleme bei der Vergärung werden wirksam vermieden, wie beispielsweise massive Schaumbildung oder plötzliche Verringerung einer Mikroorganismenpopulation bzw. Verringerung der Mikroorganismenaktivität.

In einer bevorzugten Ausführungsform der Erfindung mit besonders guter bioenzymatischer Aktivität ist in dem Enzympräparat A die Xylanase eine Endo-1,4-Xylanase, die Amylase eine Glucoamylase, die Lipase eine Triacylglycerin-Lipase und/oder die Glucanase eine Endo-1,3-beta-Glucanase.

In einer weiteren bevorzugten Ausführungsform der Erfindung mit besonders guter bioenzymatischer Aktivität ist in dem Enzympräparat B die Protease ein Subtilisin.

Eine besonders einfach herzustellende, erfindungsgemäße Mehrkomponenten-Zubereitung erzielt man dadurch, dass die Mehrkomponenten-Zubereitung die Spurennährstoffmischung A, das Enzympräparat A und das Enzympräparat B in jeweils gleichen Volumen-Anteilen enthält.

Eine besonders hohe Wirksamkeit der erfindungsgemäßen Mehrkomponenten-Zubereitung erzielt man dadurch, dass die Mehrkomponenten-Zubereitung die Spurennährstoffmischung A, das Enzympräparat A und das Enzympräparat B in den Volumen-Anteilen 1:2:1 enthält.

Ferner ist es bei einem Verfahren der o.g. Art erfindungsgemäß vorgesehen, dass dem Fermenter zusätzlich eine Mehrkomponenten-Zubereitung gemäß der zuvor beschriebenen Art zugegeben wird.

Dies hat den Vorteil, dass ein stabiler Verfahrensablauf im Fermenter über einen langen Zeitraum hinweg ohne Havarien beispielsweise durch massive Schaumbildung auch bei Zugabe unterschiedlichster organischer Reststoffe während des laufenden Vergärungsprozesses und eine gleichzeitige Verbesserung der Aktivität von beteiligen Mikroorganismen und eine dadurch bedingte Erhöhung der Biogasausbeute pro Zeiteinheit erzielt wird.

Eine weitere Verbesserung des Vergärungsprozesses hinsichtlich Stabilität, Vermeidung von Schaumbildung und Erhöhung des Biogasertrages erzielt man dadurch, dass dem Fermenter zusammen mit dem initialen Einbringen der organischen Reststoffe von der Spurennährstoffmischung A 170 bis 250 ml, insbesondere 200 ml, je Tonne organische Reststoffe, von dem Enzympräparat A 170 bis 250 ml, insbesondere 200 ml, je Tonne organische Reststoffe und von dem Enzympräparat B 170 bis 250 ml, insbesondere 200 ml, je Tonne organische Reststoffe zugegeben werden.

Eine weitere Verbesserung des Vergärungsprozesses hinsichtlich Stabilität, Vermeidung von Schaumbildung und Erhöhung des Biogasertrages erzielt man dadurch, dass dem Fermenter ab dem 2. Tag pro Tag 15 ml bis 25 ml, insbesondere 20 ml, je Tonne des organischen Reststoffes der Spurennährstoffmischung A, 30 ml bis 45 ml, insbesondere 40 ml, je Tonne des organischen Reststoffes des Enzympräparats A und 15 ml bis 25 ml, insbesondere 20 ml, je Tonne des organischen Reststoffes des Enzympräparats B zugegeben werden.

Eine weitere Optimierung des Vergärungsprozesses erzielt man dadurch, dass die organischen Reststoffe mit einem Trockensubstanzgehalt von 3% bis 90%, insbesondere 18%, 20%, 60% oder 87%, zugegeben werden. Dieser Wert hängt von den eingesetzten Reststoffen ab. Er kann speziell für organische Abfallstoffe (z.B. aus der Lebensmittelindustrie) in einem Bereich zwischen 3 % und etwa 60 % liegen, im Mittel ca. bei 20 %. In der Landwirtschaft ist die Spanne weiter, da hier auch Stroh zu Einsatz kommt mit bis zu 87 % TS (Trockensibstanz).

Einen kontinuierlichen Ablauf der Vergärung über einen längeren Zeitraum von Wochen bis Monate hinweg erzielt man dadurch, dass weitere organische Reststoffe täglich in einer der initialen Menge entsprechenden Menge zugegeben werden.

Für eine optimale Aktivität und Lebensdauer, der den Vergärungsprozess vorantreibenden Bakterien wird, der Fermenter bei einer Temperatur von 36°C bis 55°C, insbesondere 40°C bis 50°C, insbesondere 45°C, betrieben.

Weitere Vorteile betreffend die erfindungsgemäße Mehrkomponenten-Zubereitung und das erfindungsgemäße Verfahren bestehen darin, dass die Abbauleistung entsprechend den Vorzügen jedes Materials gefördert wird. Auch die Laufzeit der Zersetzung von Abfällen und die Zeiten zum Hochfahren eines Systems werden verbessert. Es ist eine Prozessoptimierung in Abfallvergärungsanlagen realisierbar, wobei der Anlagenbetrieb stabilisiert und eine verbesserte Abbaurate erzielt werden. Eine derart optimierte Anlage ist weniger anfällig für kostspielige Störungen/Havarien und kann die zu verwertenden Abfälle zügig mit einer höheren Ausbeute zu Biogas umwandeln.

Die Erfindung wird im Folgenden anhand der Zeichnung näher erläutert. Diese zeigt in
- Fig. 1: die bekannte Methanogenese der sogenannten "anaeroben Nahrungskette" in einem schematischen Blockdiagramm.

Die erfindungsgemäße Mehrkomponenten-Zubereitung beinhaltet in Kombination als Komponenten eine Spurennährstoffmischung A, ein Enzympräparat A und ein Enzympräparat B.

Erfindungsgemäß ist die kombinierte Anwendung von drei Komponenten vorgesehen, so dass der Prozess der Biogaserzeugung effizienter und störungsfrei abläuft. Jede dieser Komponenten allein kann diese Wirkung nicht erzielen oder sogar negative Effekte haben. Die Komponenten werden erfindungsgemäß zwar in Kombination verwendet, jedoch wegen ggf. negativer Wechselwirkungen nicht vor der Zugabe direkt miteinander vermischt, sondern werden einzeln dem Fermenter zugeben. In der dann geringeren Konzentration kommt es nicht zu negativen Wechselwirkungen zwischen den Komponenten.

Die Spurennährstoffmischung A (die erste Komponente) hat für sich betrachtet folgende Bestandteile, 25 bis 50 Gew.-% Dinatrium-Nickel-EDTA, 5 bis 10 Gew.-%, Cobaltdinatriumethylendiamintetraacetat und 2,5 Gew.-%Natriumselenit und 37,5 bis 67,5 Gew.-% Wasser.

Der Ausdruck EDTA bezeichnet hierbei Ethylen-Diamin-Tetraessigsäure. Dinatrium-Nickel-EDTA ist eine chemische Verbindung, die aus Nickel, dem Chelatbildner EDTA (Ethylen-Diamin-Tetraessigsäure) und Dinatriumionen besteht. Es handelt sich um ein komplexes Salz, das Nickel in chelatierter (gebundener) Form enthält.

Das Enzympräparat A (die zweite Komponente) hat für sich betrachtet folgende Bestandteile, 5 Gew.-% einer Xylanase insbesondere Endo-1,4-Xylanase, 5 bis 10 Gew.-% einer Amylase, insbesondere Glucoamylase, 2,5 Gew.-% einer Lipase, insbesondere Triacylglycerin-Lipase, 5 bis 10 Gew.-% einer Cellulase, 1,0 bis 2,0 Gew.-% einer Glucanase, insbesondere Endo-1,3-beta-Glucanase, 0,1 Gew.-% Dextrose, 0,01 Gew.-% Natriumbenzoat, 0,02 Gew.-% Sorbat und 70,37 bis 81,37 Gew.-% Wasser.

Das Enzympräparat B (die dritte Komponente) hat für sich betrachtet folgende Bestandteile, 2,5 Gew.-% einer Protease, insbesondere Subtilisin, 1,9 Gew.-% Dextrose, 0,24 Gew.-% Natriumbenzoat, 0,48 Gew.-% Sorbat und 94,88 Gew.-% Wasser

Die Dosierung und die Art der Additive bzw. Komponenten haben einen großen Einfluss auf die Wirksamkeit im Prozess, die hauptsächlich vom Substrat (organische Reststoffe) abhängt. Daher ist es wichtig, Additive bzw. Komponenten besser zu nutzen, um die Abbauleistung entsprechend den Vorzügen jedes Materials zu fördern. Auch die langsame Zersetzung von Abfällen und die langen Zeiten zum Hochfahren eines Systems von bis zu 28 Tagen geben Anlass zur Weiterentwicklung der Technologie.

In einem Praxistest wurde folgender Versuchsaufbau und Versuchsablauf realisiert:
Der Praxistest wurde in einer Abfallverwertungsanlage mit folgenden Eckdaten durchgeführt:
- täglich 110-115 t Lebensmittelabfälle mit 18 % Trockensubstanzgehalt (TS)
- 1.000 m³/h Rohgas mit 61% CH4
- 2 parallel betriebene Fermenter mit jeweils 3.000 m³ (5 Gew.-% TS, 45 °C)
- 55 Tage Verweilzeit in den Fermentern
- 6.000 mg/l NH4-N -> 1.000 NH3 mg/l, d.h. Ammoniak Hemmung vorliegend
- Laboranalyse des Fermenter Inhaltes zeigt einen etwas niedrigen Gehalt an Nickel

Hierbei konnte während des Ablaufs des herkömmlichen Vergärungsvorgariges folgendes beobachtet:
Es kam zu einer massiven Schaumbildung (teilweise sehr plötzlich, ganz kleine weiße Blasen, stabile Struktur), die für kostspielige Havarien sorgt. Bei Probeentnahme in einem 30 Liter Eimer bildet sich der Schaum weiterhin in den folgenden Stunden. Bisher konnte keine technische oder biologische Lösung für dieses Problem gefunden werden
Aus diesen Beobachtungen wurden folgende Annahmen abgeleitet:
Makromoleküle (vor allem Eiweiß und Fette) begünstigen die Schaumbildung. Die beteiligten Mikroorganismen in Form von Bakterien leiden durch 5-tach zu hohe Ammoniakkonzentration und knappe Nickelkonzentration unter starkem Selektionsdruck und reagieren darauf durch Schutzmechanismen und eine schlechtere Performance, was ebenfalls schaumfördernd wirkt.

Zur Vermeidung der o.g. Probleme während des Verfahrensablaufes wurden folgende Maßnahmen getroffen:

In einem Zeitraum von 8 Wochen wurden verschiedene Additive in einen der beiden Fermenter der Anlage dosiert. Zur Überprüfung wurden regelmäßig aus beiden Fermentern Proben genommen und bezüglich der Schaumbildung optisch bewertet.

Es wurden folgende drei Additive bzw. Komponenten in Kombination verwendet:
(1) Eine Spurennährstoffmischung A für sich betrachtet enthaltend 25-50 Gew.-% Dinatrium-Nickel-EDTA, 5-10 Gew.-% Cobaltdinatriumethylendiamintetraacetat und 2,5 Gew.-% Natriumselenit und 37,5 bis 67,5 Gew.-% Wasser.
(2) Ein Enzympräparat A enthaltend 5 Gew.-% Endo-1,4-Xylanase, 5-10 Gew.-% Glucoamylase, 2,5 Gew.-% Triacylglycerin-Lipase und 5-10 Gew.-%Cellulase, 1,0-2,0 Gew.-% Endo-1,3-beta-Glucanase, 0,1 Gew.-% Dextrose, 0,01 Gew.-% Natriumbenzoat, 0,02 Gew.-% Sorbat und 70,37 bis 81,37 Gew.-% Wasser. Diese Enzymkomplexe sind speziell auf fett-, stärke- und cellulosehaltige Inputstoffe gerichtet.
(3) Ein Enzympräparat B enthaltend 2,5 Gew.-% Subtilisin, 1,9 Gew.-% Dextrose, 0,24 Gew.-% Natriumbenzoat, 0,48 Gew.-% Sorbat und 94,88 Gew.-% Wasser. Dieser Enzymkomplex dient dem Aufschluss von Proteinen.

Von der Spurennährstoffmischung A wurden dem Fermenter 20 Liter sofort und ab dem 2. Tag 2,0 Liter pro Tag zugegeben.

Von dem Enzympräparat A wurden dem Fermenter 20 Liter sofort und ab dem 2. Tag 4,0 Liter pro Tag zugegeben.

Von dem Enzympräparat B wurden dem Fermenter 20 Liter sofort und ab dem 2. Tag 2,0 Liter pro Tag zugegeben.

In dem Fermenter mit den obigen Zugaben ergaben sich über den Versuchszeitraum folgende Ergebnisse:
Überraschenderweise konnte bereits nach nur einer Woche eine deutliche Verbesserung bezüglich der Schaumbildung festgestellt werden. In der Regel benötigt so ein komplexes System mehrere Wochen, um sich an Änderungen anzupassen.

Nach drei Wochen konnte eine Homogenisierung des Fermenterinhaltes beobachtet werden. Der Gärbrei wurde deutlich geschmeidiger und weniger schleimig, was auf eine verbesserte Viskosität hinweist. Zudem wurden auch Ablagerungen in einem Ausweichbehälter und in den Rohrleitungen aufgelöst.

Nach etwa vier Wochen kam es zu einem Anstieg des Schwefelwasserstoffes im Biogas, was durch Zugabe eines Entschwefelungsmittels behandelt wurde. Der plötzliche Anstieg kann auf die Auflösung einer Sinkschicht im Fermenter zurückgeführt werden.

Zusammenfassend kann festgestellt werden, dass nicht nur die Schaumbildung in extrem kurzer Zeit sehr deutlich verringert wurde, sondern auch der Abbau insgesamt stark verbessert wurde, sodass selbst alte Ablagerungen aufgelöst wurden.

Die Kombination aus den drei Zusatzstoffen Spurennährstoffmischung A (30 ml je t Substrat), Enzympräparat A (60 ml je t Substrat) und Enzympräparat B (30 ml je t Substrat) hat sich bewährt. Durch den kombinierten Einsatz der drei Additive konnten SSynergieeffekte erzielt werden, die so ment zu erwarten waren.

Als Wirkmechanismen, welche die überraschende, vorteilhafte Wirkung erzielen, weruen folgende Prozesse und Abläufe vermutet:
Spurenelemente sind als Additive essenziell für die Gewährleistung eines stabilen Vergärungsprozesses und können zur Steigerung der Biogasproduktion eingesetzt werden. Sie sind die wichtigen Cofaktoren von Enzymen, die an der Methanproduktion beteiligt sind. Mit der für Abfallvergärungsanlagen angepassten Spurennährstoffmischung A wird eine sehr gut abgestimmte Mischung der wichtigsten Spurenelemente in einem biologisch verfügbarem Chelatkomplex genutzt. So können diese von den Mikroorganismen am besten aufgenommen werden. Die Spurennährstoffmischung A allein bewirkt offenbar erst einmal eine Stabilisierung des Prozesses, da vorliegende Mängel beseitigt werden und sich die Mikroorganismen (Bakterien) besser vermehren können. Die beiden Enzympräparate A und B übernehmen die Aufgabe die sonst langsam ablaufende Hydrolyse stark zu beschleunigen, was einen besseren Abbau, eine verbesserte Versorgung der Mikroorganismen, deren stärkere Vermehrung und damit auch eine höhere Produktion an Biogas nach sich zieht.

Enzympräparat A ist eine speziell für den Abfallbereich entwickelte Kombination von hydrolytischen Enzymen (Endo-1,4-Xylanase, Glucoamylase, Triacylglycerin-Lipase, Cellulase). Diese beschleunigen den Abbau von verschiedenen Stoffen um ein Vielfaches, darunter Cellulose, Hemicellulose, Stärke, Öle, Fette, Beta-Glucane und Pektine. Mit Enzympräparat B, einer Protease, wird das Zusammenspiel der Additive abgerundet. Dieses Enzympräparat sorgt für eine Spaltung der Eiweißstrukturen und setzt dabei Aminosäuren frei. Die Kombination der beiden sehr breit aufgestellten, spezifischen Enzymmischungen sorgt auch bei stark schwankenden Substrat-Arten bzw. -qualitäten (organische Reststoffe) für eine gleichbleibend schnelle Zersetzung.

Durch den schnellen Abbau wird eine große Menge gut löslicher kleiner Moleküle (verschiedene Zucker, Fettsäuren und Aminosäuren) freigesetzt, welche wiederum von den Mikroorganismen weiter verstoffwechselt werden. Diese leichter zugängliche Nahrung sorgt für eine Vermehrung der Mikroorganismen (Bakterien). Dies kann schnell zu Übersäuerungen führen, da die Säurebildung gesteigert wird und die sich nur langsam vermehrenden, methanbildenden Bakterien dies aufholen müssen. Dafür müssen diese deren Stoffwechselaktivität erhöhen und sich stärker vermehren. Der dadurch wiederum steigende Bedarf der Mikroorganismen an Spurennährstoffen wird durch die Spurenelemente aus Spurennährstoffmischung A gedeckt, sodass sich ein Gleichgewicht auf hohem Niveau einstellt. Dies macht die gesamte Biogasanlage produktiver und sorgt für einen stabilen Betrieb. Da besonders die Makromoleküle (z.B. Eiweiß, Stärke, Pektine usw.) zu schwerwiegenden Problemen wie extremer Schaumbildung beitragen, ist durch deren gesteigerten Abbau auch in Bezug darauf vorgebeugt.

## Patentansprüche

1. Mehrkomponenten-Zubereitung für die Zugabe beim Vergären von organischen Reststoffen in ein Fermentermedium, insbesondere unter anaeroben Bedingungen, insbesondere zum Erzeugen von Biogas,
**gekennzeichnet durch,**
folgende Inhaltsstoffe,
eine Spurennährstoffmischung A, die für sich betrachtet folgendes beinhaltet, 25 bis 50 Gew.-% Dinatrium-Nickel-EDTA, 5 bis 10 Gew.-%, Cobaltdinatriumethylendiamintetraacetat, 2,5 Gew.-%Natriumselenit und 37,5 bis 67,5 Gew.-% Wasser;
ein Enzympräparat A, das für sich betrachtet folgendes beinhaltet, 5 Gew.-% einer Xylanase, 5 bis 10 Gew.-% einer Amylase, 2,5 Gew.-% einer Lipase, 5 bis 10 Gew.-% einer Cellulase, 1,0 bis 2,0 Gew.-% einer Glucanase, 0,1 Gew.-% Dextrose, 0,01 Gew.-% Natriumbenzoat, 0,02 Gew.-% Sorbat und 70,37 bis 81,37 Gew.-% Wasser;
ein Enzympräparat B, das für sich betrachtet folgendes beinhaltet, 2,5 Gew.-% einer Protease, 1,9 Gew.-% Dextrose, 0,24 Gew.-% Natriumbenzoat, 0,48 Gew.-% Sorbat und 94,88 Gew.-% Wasser.

2. Mehrkomponenten-Zubereitung nach Anspruch 1, **dadurch gekennzeichnet, dass** in dem Enzympräparat A die Xylanase eine Endo-1,4-Xylanase, die Amylase eine Glucoamylase, die Lipase eine Triacylglycerin-Lipase und/oder die Glucanase eine Endo-1,3-beta-Glucanase ist.

3. Mehrkomponenten-Zubereitung nach Anspruch 1 oder 2, **dadurch gekennzeichnet, dass** in dem Enzympräparat B die Protease ein Subtilisin ist.

4. Mehrkomponenten-Zubereitung nach mindestens einem der vorhergehenden Ansprüche, **dadurch gekennzeichnet, dass** die Mehrkomponenten-Zubereitung die Spurennährstoffmischung A, das Enzympräparat A und das Enzympräparat B in jeweils gleichen Volumen-Anteilen enthält.

5. Mehrkomponenten-Zubereitung nach mindestens einem der vorhergehenden Ansprüche, **dadurch gekennzeichnet, dass** die Mehrkomponenten-Zubereitung die Spurennährstoffmischung A, das Enzympräparat A und das Enzympräparat B in den Volumen-Anteilen 1:2:1 enthält.

6. Verfahren zum Erzeugen von Biogas, wobei organische Reststoffe in einen Fermenter gegeben und dort anaerob vergärt werden,
**dadurch gekennzeichnet,**
**dass** dem Fermenter zusätzlich eine Mehrkomponenten-Zubereitung gemäß wenigstens einem der vorhergehenden Ansprüche zugegeben wird.

7. Verfahren nach Anspruch 6, **dadurch gekennzeichnet, dass** dem Fermenter zusammen mit dem initialen Einbringen der organischen Reststoffe von der Spurennährstoffmischung A 170 bis 250 ml, insbesondere 200 ml, je Tonne organische Reststoffe, von dem Enzympräparat A 170 bis 250 ml, insbesondere 200 ml, je Tonne organische Reststoffe und von dem Enzympräparat B 170 bis 250 ml, insbesondere 200 ml, je Tonne organische Reststoffe zugegeben werden.

8. Verfahren nach Anspruch 6 oder 7, **dadurch gekennzeichnet, dass** dem Fermenter ab dem 2. Tag pro Tag 15 ml bis 25 ml, insbesondere 20 ml, je Tonne des organischen Reststoffes der Spurennährstoffmischung A, 30 ml bis 45 ml, insbesondere 40 ml, je Tonne des organischen Reststoffes des Enzympräparats A und 15 ml bis 25 ml, insbesondere 20 ml, je Tonne des organischen Reststoffes des Enzympräparats B zugegeben werden.

9. Verfahren nach mindestens einem der Ansprüche 6 bis 8, **dadurch gekennzeichnet, dass** die organischen Reststoffe mit einem Trockensubstanzgehalt von 3% bis 90%, insbesondere 18%, 20%, 60% oder 87%, zugegeben werden.

10. Verfahren nach mindestens einem der Ansprüche 6 bis 9, **dadurch gekennzeichnet, dass** weitere organische Reststoffe täglich in einer der initialen Menge entsprechenden Menge zugegeben werden.

11. Verfahren nach mindestens einem der Ansprüche 6 bis 10, **dadurch gekennzeichnet, dass** der Fermenter bei einer Temperatur von 36°C bis 55°C, insbesondere 40°C bis 50°C, insbesondere 45°C, betrieben wird.
